# EUROPEAN PATENT SPECIFICATION

(11) **EP 4 329 743 B1**
(45) Date of publication and mention of the grant of the patent: **11.03.2026**
(21) Application number: 21722781.8
(22) Date of filing: 26.04.2021
(51) Int. Cl.: A61K 31/22, A61K 45/06, A61K 9/00, A61P 31/12, A61P 31/14, A61P 31/16, A61P 31/18, A61P 31/20, A61P 31/22

(54) **ETHYL PYRUVATE FOR USE IN THE TREATMENT OR PROPHYLAXIS OF INFLUENZA**
ETHYLPYRUVAT ZUR BEHANDLUNG ODER VORBEUGUNG VON INFLUENZA
PYRUVATE D'ÉTHYLE POUR SON UTILISATION DANS LE TRAITEMENT OU LA PROPHYLAXIE DE LA GRIPPE

(43) Date of publication of application: 06.03.2024
(73) Proprietor: Birkenmeier, Gerd, 04103 Leipzig (DE)
(72) Inventor: Birkenmeier, Gerd, 04103 Leipzig (DE)
(74) Representative: Kraus & Lederer PartGmbB
(86) International application number: PCT/EP2021/060830
(87) International publication number: WO 2022/228651

(56) References cited:
- CN-A- 106 727 320
- TANWAR OMPRAKASH ET AL: "Ethyl Pyruvate as a Potential Defense Intervention against Cytokine Storm in COVID-19?", ACS OMEGA, vol. 6, no. 11, 23 March 2021 (2021-03-23), US, pages 7754 - 7760, XP055872086, ISSN: 2470-1343, DOI: 10.1021/acsomega.1c00157
- FINK M. P.: "Ethyl pyruvate: a novel anti-inflammatory agent", JOURNAL OF INTERNAL MEDICINE, vol. 261, no. 4, 1 April 2007 (2007-04-01), GB, pages 349 - 362, XP055872111, ISSN: 0954-6820, DOI: 10.1111/j.1365-2796.2007.01789.x
- FINK MITCHELL: "Ethyl Pyruvate: A Novel Treatment for Sepsis", CURRENT DRUG TARGETS, vol. 8, no. 4, 1 April 2007 (2007-04-01), US, pages 515 - 518, XP055872112, ISSN: 1389-4501, DOI: 10.2174/138945007780362791
- YANG R ET AL: "Ethyl pyruvate is a novel anti-inflammatory agent to treat multiple inflammatory organ injuries", JOURNAL OF INFLAMMATION, vol. 13, no. 1, 3 December 2016 (2016-12-03), XP055872090, DOI: 10.1186/s12950-016-0144-1
- FINK ET AL: "Ringer's ethyl pyruvate solution: a novel resuscitation fluid for the treatment of hemorrhagic shock and sepsis", JOURNAL OF TRAUMA, INJURY, INFECTION, AND CRITICAL CARE, WILLIAMS AND WILKINS, BALTIMORE, MD, US, vol. 54, no. 5 suppl, 1 January 2003 (2003-01-01), pages S141 - S143, XP008126780, ISSN: 1079-6061
- VYAWAHARE N S ET AL: ""The Future Magic Bullet": A Review of Pharmacological Activities of Ethyl Pyruvate and its Derivatives", CURRENT DRUG THERAPY, vol. 7, no. 2, 1 June 2012 (2012-06-01), NL, pages 144 - 149, XP055872084, ISSN: 1574-8855, DOI: 10.2174/157488512800676048

## Description

### FIELD OF THE INVENTION

The present invention relates to ethyl pyruvate for use in the treatment or prophylaxis of influenza. Further envisaged is a corresponding pharmaceutical composition for use in the treatment or prophylaxis of influenza.

### BACKGROUND OF THE INVENTION

Viruses are small non-living particles consisting of DNA or RNA covered by membranes. Viruses due to specific membrane proteins are capable to enter host cells for replication and to thus infect hosts. Virus infections cause annual epidemics and occasional pandemics which are associated with 200,000 to 500,000 death each year. Influenza, for example, produced direct costs due to lost productivity and associated medical treatment and, in the US alone, responsible for a total cost of over $ 10 Billion per year.

Viruses are non-living entities and as such do not inherently have their own metabolism. Upon entering cells via receptor-ligand interaction viruses replicate by exploiting the energy-supplying machinery of the host cells. This leads to an activation of metabolic pathways such as glycolysis (Sanchez and Lagunov, Virology,2015, 479-480, 609-618). Inhibition of glycolysis therefore would prevent or at least suppress viral maturation in mamma cells. Possible targets are glyoxalase-1 and/or pyruvate kinase (Birkenmeier et al., PLoS One, 2016, 11, 1-19; Worku et al., PLoS One, 2015, 10, 1-19).

Viruses such as influenza virus are primarily transmitted from person to person via large virus-laden droplets that are generated when infected persons speak, cough or sneeze. Transmission can also occur through direct contact with respiratory secretions contaminating surfaces. Adults might be able to spread influenza to others from day 1 before getting symptoms to approx. 5 days after symptoms start.

Influenza viruses belong to the family Orthomyxoviridae and one of their major characteristics is the rapid rate of viral evolution. They are categorized into four types: A, B, C and D. Influenza A viruses circulate in many avian species and infect several mammalian species including humans. Influenza B viruses infect only humans, and influenza C viruses infects humans and pigs. Influenza D viruses primarily affect cattle and are not known to infect or cause illness in humans (Sautto et al., Virol J., 2018, 15(1), 17).

Influenza A viruses, together with influenza B viruses, are responsible for human seasonal epidemics and pre-pandemic outbreaks. Each influenza virus is further classified, based on the surface glycoproteins hemagglutinin (HA) and neuraminidase (NA), into subtypes. At present, 20 different HA subtypes and 11 NA subtypes have been identified circulating in birds and mammals.

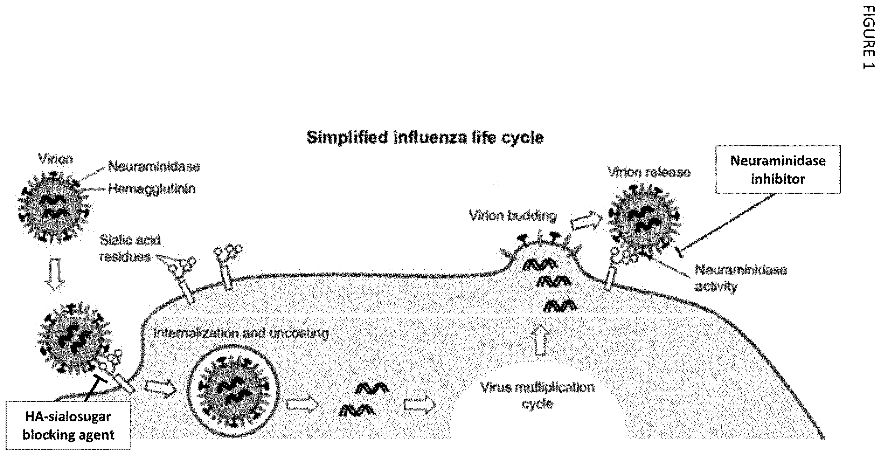

HA is a lectin that mediates binding of the virus to and entry of the viral genome into the target cell (Suzuki, Biol Pharm Bull, 2005, 28(3), 399-408). In contrast, neuraminidase is an enzyme involved in the release of progeny virus from infected cells, by cleaving sugars that bind the mature viral particles. Both HA and NA, are responsible for the binding to sialic acid, the receptor on target cells (see also Fig. 1).

Owing to the pivotal role of HA in the viral life cycle, as well as its exposition on the viral envelope, this protein is the primary neutralizing target of the humoral immune response. HA is a trimeric protein on the outer membrane of the virus. HA is proteolytically processed by proteases into HA1 and HA2. While HA1 binds to the receptor sialic acid on the surface of host cells, HA2 mediates cell membrane fusion.

HA initiates infection by binding to the sialic acid mostly N-acetyl-neuraminic acid (Neu5Ac) acting as cell surface receptors and by inducing fusion between viral and cellular membranes. Virus binding depends not only on HA affinity for the terminal sialic acid residues, but also on the structure of the underlying oligosaccharide and protein or lipid moieties of the receptors (Matrosovich, Top Curr Chem, 2015, 367,1-28).

Sialic acid can be acetylated at position 4, 7, 8 and 9. In natural glycoconjugates, sialic acids are α2-3- or α2-6-linked to Gal and GalNAc, α2-6-linked to GlcNAc, or α2-8-linked to the second sialic acid residue. Influenza viruses generally do not bind to α2-8-linked Neu5Ac and can recognize only α2-3- or α2-6-linked sialic acid moieties such as Neu5Acα2-3/6Gal, Neu5Acα2-3/6GalNAc, and Neu5Acα2-6GlcNAc. Thus, human influenza viruses preferentially bind to α2-6-linked sialic acids (Neu5Acα2-6Gal), whereas avian influenza viruses preferentially recognize sialic acid linked to galactose by an α(2-3) linkage (Neu5Acα2-3Gal) (Connor et al., Virology, 1994, 205(1):17-23).

Influenza C viruses that cause mild respiratory infections in humans differ from other influenza viruses because their preferred sialic acid is N-acetyl-9-O-acetylneuraminic acid (Neu5,9Ac2), the presence of an acetylesterase rather than a neuraminidase as well as the presence of the hemagglutinin-esterase-fusion (HEF) protein (Rosenthal et al., Nature, 1998, 396(6706), 92-6). The novel swine-origin H1N1 pandemic influenza virus A binds exclusively to α2-6-linked sialyl sequences (Bradley et al., Virology, 2011, 413(2), 169-82). Influenza D virus diverges from its related influenza C virus in the recognition of 9-O-acetylated N-acetyl- or N-glycolyl-neuraminic acid-containing glycan receptors (Liu et al., Virology, 2020, 545, 16-23). Avian influenza viruses preferentially recognize sialosugar chains (i.e. sialic acid containing sugar chains) terminating in sialic acid-α2,3-galactose (SAα2,3Gal), whereas human influenza viruses preferentially recognize SAα2,6Gal.

Introduction of new hemagglutinin (HA) subtypes by mutation is believed to lead to conversion of the sialosugar preference which can lead to pandemics. For example, the highly pathogenic avian influenza A (H5N1) virus has caused severe acute respiratory infection in humans, with a fatality rate of more than 50% (Zeng et al., J Virol, 2019, 93, 10).

Next to HA, the neuraminidase (NA) is a type II membrane protein that is present in homotetrameric form in the viral envelope. NA allows the virus to penetrate the mucus layer coating the respiratory epithelium and it promotes virus release and prevents clumping of virions by removing receptors enzymatically from the cell surface and viral glycoproteins (Palese et al., Virology, 1974, 61(2), 397-410).

Sialic acid linked to glycoproteins and gangliosides is used not only by influenza viruses as a receptor for cell entry but also by other viruses including important human and animal pathogens such as members of Paramyxoviridae, Caliciviridae (Norovirus), Picornaviridae, Reoviridae, Polyomaviridae, Adenoviridae, Adenoviridae and Parvoviridae (Matrosovich, Top Curr Chem, 2015, 367,1-28).

Variations in the sialic acid receptor specificity are important determinants for host range, tissue tropism, pathogenicity, and transmissibility of these viruses. For example, Corona viruses use the homotrimer spike (S) glycoprotein to promote host cell attachment to the sialylated angiotensin-converting enzyme-2 (ACE-2) receptor and fusion of viral and cellular membrane for entry. Surprisingly, corona viruses use also 9-O-acetyl-sialic acid (9-O-Ac-Sia) as a receptor which is terminally linked to oligosaccharides decorating glycoproteins and gangliosides including the ACE-2 glycoprotein at the host cell surface. O-acetylated SAs interact with the lectin-like spike glycoprotein of SARS CoV-2 for the initial attachment of viruses to enter the host cells. In addition, SARS-CoV-2 hemagglutinin-esterase (HE) acts as the classical glycan-binding lectin and receptor-degrading enzyme (Kim, Int J Mol Sci, 2020, 21(12), 4549).

Thus, not only the functional activity of the entry receptors on the host cells but also the extent and type of their glycan chains, in particular their sialic acids, exposed at the cell surface may influence and modulate the rate of infection of the SARS-CoV-2, as demonstrated for other members of this virus family (Schwegmann-Wessels and Herrler, Glycoconj J, 2006, 23(1-2), 51-8).

It is assumed that partly because of the selection pressure the expression of sialic acids is highly regulated. Different cell and tissue types show marked differences in abundance, types, and linkages of sialic acids. Approximately 20 different sialyltransferases attach sialic acid to glycans via α2-3, α2-6, or α2-8 linkages. The human influenza A and B viruses are among rare examples of pathogens with known preference for α2-6-linked sialic acid. Types of linkage preference are also shared by human pathogens or commensals such as human coronavirus OC43, polyoma virus JC, adeno-associated virus serotype 5, and Streptococcus mutans.

The influenza hemagglutinin (HA) is the major target of host protective antibodies. This protein undergoes continuous genetic and antigenic evolution, resulting in the emergence of several phylogenetically and geographically distinct clades and subclades. The Hemagglutinin (HA0) is post translationally cleaved into HA1 and HA2 subunits that activate the membrane fusion potential of the HA and as such serves as a necessary step for virus infectivity. Most avian influenza viruses possess a single arginine residue at the HA cleavage site. However, several animal passages result in the acquisition of multiple basic amino acids at the HA cleavage site, which permits HA0 cleavage by ubiquitously expressed intracellular proteases, including furin-like and trypsin-like proteases, enabling dissemination of virus beyond respiratory tract tissues (Palladiono et al., J Virol, 1995, 69(4), 2075-81). Therefore, HA is also an attractive molecule for the development of prophylactic and therapeutic approaches.

Current treatment options for sialosugar binding viruses such as influenza virus include vaccination, chemotherapy, or chemoprophylaxis with antiviral medication. Vaccination against influenza is often recommended for high-risk groups, such as children and the elderly, or for people with asthma, diabetes, or heart diseases. The vaccine must be reformulated each season for a specific influenza strain. Due to the high mutation rate of the virus, a particular vaccine may confer protection for no more than a few years, because over time different strains become dominant. The influenza virus is RNA virus. Because of the absence of RNA-proof reading enzymes nucleotide insertion errors often occur. In general, the virus has two major mechanisms for antigenic change: antigenic drift and antigenic shift. While both mechanisms allow to evade natural immunity, they also interfere with successful vaccination (Davies and Grilli, Epidemiol Infect, 1989, 102(2), 325-33). Antigenic drift is the process by which minor changes are introduced into key viral epitopes through point mutations in the viral genome. The second form of antigenic change that influenza viruses have at their disposal is antigenic shift. Rather than the gradual changes seen with antigenic drift, antigenic shift results in a complete exchange of HA and/or NA genes (Webster and Govorkova, Ann N Y Acad Sci, 2014, 1323(1),115-39). Antigenic shift only occurs among influenza A viruses due to their extensive animal reservoirs, the sources of antigenically distinct viruses. Any vaccination approach that targets the classic neutralizing responses to HA and/or NA must deal with antigenic drift and shift effectively.

Both influenza A and B viruses, two genera of the Orthomyxoviridae family, are the cause of substantial morbidity and mortality in humans and are targets for the current influenza vaccines. Two subtypes classified based on the antigenic properties of their surface glycoproteins HA and NA of influenza A viruses, A(H1N1) and A(H3N2), currently circulate in humans. A hallmark of influenza virus antigenic drift in humans is the replacement of older viruses by new drifted variants, although at rates dependent on subtype. Antigenically variant populations have been shown to co-circulate for longer periods in A(H1N1) and in influenza B viruses, whereas turnover is more rapid with A(H3N2) viruses. Any vaccination approach that targets the classic neutralizing responses to HA and/or NA must deal with antigenic drift effectively. It is also possible that novel vaccines that increase the potency of immunity to other viral proteins may lead to more rapid antigenic evolution.

Antiviral drugs such as the neuraminidase inhibitors oseltamivir (Tamiflu) and zanamivir (Relenza)(Chen et al., Open Forum Infect Dis, 2020, 8(1), ofaa562) as well as ion channel inhibitors (M2 inhibitors) such as amantadine and rimantadine (Mandour et al., Drug Des Devel Ther, 2018, 12, 1019-1031) have been used to increase the arsenal of treatment options against both seasonal and pandemic influenza. However, the latter induced significant gastrointestinal adverse effects (Jefferson et al., Cochrane Database Syst Rev, 2002, (3), CD001169). Endonuclease inhibitors interfere with viral RNA transcription and block virus replication in both influenza A and B viruses. Baloxavir inhibits transcription by preventing the virus from commandeering the host cell's manufacturing facilities (Yang, Ann Pharmacother, 2019, 53(7), 754-759). However, these drugs have limited efficacy when administered late and yield emergence of resistant viral strains disregarding associated site effects, such as nausea, vomiting, psychiatric effects and renal events.

Document CN106727320A discloses a composition for repairing schneiderian membrane, reduing allergic reaction, antiinflammatory action. Tanwar et al., 2021, ACS Omega, 6, 7754-7760 discloses ethyl pyruvate as potential defense intervention against cytokine storm in COVID-19.

Antibodies represent classes of protecting agents and passive transfer of serum from convalescent patients was used successfully during previous influenza pandemics. However, this approach has limited potential for implementation on a global scale due to (i) restricted supply, (ii) high risk of toxicity, (iii) high lot-to-lot variation, (iv) uncertain dosing and (v) difficulties of administration.

Thus, there is an unmet need for treatment options for infections caused by sialic acid-containing sugar chain binding viruses, in particular influenza viruses.

### OBJECTS AND SUMMARY OF THE INVENTION

The present invention addresses this need and provides the compound ethyl pyruvate for use in the treatment or prophylaxis of influenza.

The present inventor has surprisingly found that the above-mentioned compound is capable of treating and preventing a disease associated with an interaction of a receptor comprising a sialic acid-containing sugar chain with a cognate binding element, in particular an influenza infection. In particular, the present inventor has surprisingly found that the compound for use according to the invention acts competitively with the N- or O-linked acetyl residues of various sialic acids or sialic acid variants. The compound for use thus prevents viruses from binding to or docking with sialic acid-containing oligosaccharides or sugar polymers. Typical binding partners are hemagglutinins (e.g. in influenza) or lectin-like surface proteins of a virus, e.g. the S1 / S2 spike protein of coronaviruses. Convincingly and in line with the above described mode of action, the competitive inhibitory effect has been shown to disappear as soon as the acetyl residue is reduced, for example, if ethyl lactate is used. In addition, the compound for use according to the invention also inhibits viral neuraminidases, which are responsible for releasing fresh viruses from a cell.

This makes the compound for use according to the present invention a very versatile und extremely helpful tool for treatment and prophylaxis of influenza infections. The compound for use further largely excludes adverse side effects. In particular, the compound for use of the invention has a cell-protective effect due to its antioxidant, anti-inflammatory and antibacterial effects. Furthermore, it shows neuroprotective effects. A further advantage is that the use of infusions with ethyl pyruvate for septic patients has not shown any side effects in clinical studies.

A further advantageous property of the compound for use of the present invention is that it is capable of inhibiting inflammation of virus-infected cells and tissues. During a disease which is associated with an interaction of a receptor comprising a sialic acid-containing sugar chain with a cognate binding element - with cognate binding elements, for example a virus infection, cells and tissue are typically destroyed, which triggers an inflammatory reaction. This reaction is further reinforced by humoral and cellular defense mechanisms. The mediators of these mechanisms are primarily inflammatory cytokines such as TNF-alpha, IL-1, and IL-6. The compound for use of the present invention is, advantageously, capable of inhibiting the release of inflammatory cytokines and thereby abolishes the damaging effects on lung tissue. This does not negatively affect the alveolar oxygen supply. The compound for use of the present invention thus has a protective function for cells and tissues.

A further advantageous property of the compound for use of the present invention is that it is capable of inhibiting virus multiplication in host cells by inhibiting the supply of energy in metabolically activated cells. Virus-activated cells, but also tumor cells, LPS-activated immune cells and cells with a high rate of glycolysis are driven into suicide by the compound for use according to the present invention. This is due to the inhibition of glyoxalase-1 and/or pyruvate kinase which is associated with accumulation of cell-toxic methylglyoxal (MGO). Methylglyoxal induces apoptosis in the affected cells. Advantageously, non-metabolically activated cells such as normal, healthy body cells, and cells that are in a postmitotic state, are not affected by this mechanism. This makes the compound for use according to the present invention a very versatile und extremely helpful tool for an antiviral treatment.

Another helpful and advantageous property of the compound for use of the present invention is its neuroprotective effect, which could be confirmed. This property is of high relevance since several causative agents of diseases which are associated with an interaction of a receptor comprising a sialic acid-containing sugar chain with a cognate binding element, in particular viruses such as Coronavirus, are assumed to reach the central nervous system (CNS) in a neuro-invasion process, probably via the nasal mucosa and lung. In the CNS, the causative agent, e.g., the virus, causes different neuronal damages including changes in gait safety, and smell and taste distortions. Since the compound for use of the invention, e.g., ethyl pyruvate, crosses the blood-brain barrier, it becomes active also in viral infection scenarios in the brain. Thus, the compound for use of the present invention is neuroprotective (Piero et al., CNS Neurosci Ther., 2017; 23(10): 798-807).

A further advantage of the compound for use of the present invention is its additional property to kill bacteria and other pathogens, such as fungi. In many cases a disease which is an interaction of a receptor comprising a sialic acid-containing sugar chain with a cognate binding element, for example a virus infection, occurs in people with immunodeficiency, tumor diseases, diabetes, cardiovascular diseases or the elderly. In these cases, but also in infection scenarios, where the subject has no pre-existing condition, bacterial superinfections may occur because of immunosuppression in the course of a viral infection. The compound for use of the present is bactericidal and further eliminates pathogenic fungi. Advantageously, non-pathogenic microbes such as Lactobacilli are not affected.

Yet another extremely helpful feature of the compound for use of the present invention is its capability to prevent virus multiplication in the intestinal tract. Since the interaction of a receptor comprising a sialic acid-containing sugar chain with a cognate binding element, e.g. during virus infection, may also occur in the intestinal mucosa, this tissue is a suitable site for replication of a causative agent, e.g. virus, which can additionally contribute to disease spreading and may also increase the disease symptoms. Since the compound for use of the present invention can be formulated as oral composition and can be ingested, e.g., via drinking, it easily reaches the infected intestinal regions, thus attacking the causative agent there as well.

An extremely useful further property of the compound for use of the present invention is its volatility. I.e., it can be administered via aerosols or the like directly to the respiratory tract or deep alveolar areas of the lung through the mouth and/or the nose. The use of such an inhalable medication is considered valuable for causative agents which are present in the respiratory tract and lungs. The compound for use of the present invention can thus be advantageously applied to all areas of the respiratory tract in variable concentrations, e.g., by using a vaporizer or a spraying device through the nose and/or mouth.

In a further aspect the present invention relates to a pharmaceutical composition comprising ethyl pyruvate for use in the treatment or prophylaxis of influenza.

In a further embodiment the pharmaceutical composition for use comprises one or more additional therapeutically active ingredients.

It is preferred that said additional therapeutically active ingredient is an antiviral compound, an anti-inflammatory compound, an anti-bacterial agent and/or analgesic.

In a further preferred embodiment, the antiviral compound is an agent which interferes with the binding of a virus to a cellular receptor, an entry inhibitor, an uncoating inhibitor, a reverse transcriptase inhibitor, an integrase inhibitor, a protease inhibitor, a viral assembly inhibitor, pyrazinecarboxamide, a neuraminidase inhibitor, an influenza polymerase domain inhibitor, an influenza CAP-binding PB2 domain inhibitor or a viral release inhibitor.

In another preferred embodiment, the antiviral compound is one or more of the group of Abacavir; Acyclovir; Adefovir; Amantadine; Ampligen; Amprenavir; Arbidol; Atazanavir; Atripla; ; Balavir; Baloxavir marboxil; Biktarvy; Boceprevir; Cidofovir; Cobicistat; Combivir; Daclatasvir; Darunavir; Delavirdine; Descovy; Didanosine; Docosanol; Dolutegravir; Doravirine; Ecoliever; Edoxudine; Efavirenz; Elvitegravir; Emtricitabine; Enfuvirtide; Entecavir; Etravirine; Famciclovir; Fomivirsen; Fosamprenavir; Foscarnet; Fosfonet; Fusion inhibitor; Ganciclovir; Ibacitabine; Ibalizumab; Idoxuridine; Imiquimod; Imunovir; Indinavir; Inosine; Integrase inhibitor; Interferon type I; Interferon type II; Interferon type III; Lamivudine; Letermovir; Lopinavir; Loviride; Maraviroc; Methisazone; Moroxydine; Nelfinavir; Nevirapine; Nexavir; Nitazoxanide; Norvir; Nucleoside analogues; Oseltamivir; Peginterferon alfa-2a; Peginterferon alfa-2b; Penciclovir; Peramivir; Pleconaril; Podophyllotoxin; Pyramidine; Raltegravir; Remdesivir; Reverse transcriptase inhibitor; Ribavirin; Rilpivirine; Rimantadine; Ritonavir; Saquinavir; Simeprevir; Sofosbuvir; Stavudine; Synergistic enhancer; Telaprevir; Telbivudine; Tenofovir alafenamide; Tenofovir disoproxil; Tenofovir; Tipranavir; Trifluridine; Trizivir; Tromantadine; Truvada; Valaciclovir; Valganciclovir; Vicriviroc; Vidarabine; Viramidine; Zalcitabine; Zanamivir; Zidovudine, and an antiviral plant extract.

In a further preferred embodiment, the antibacterial agent is one or more of the group of penicillin, aminoglycoside, tetracycline, macrolide, fluoroquinolone, and sulfonamide.

In a further preferred embodiment said antibacterial agent is one or more of the group of penicillin, aminoglycoside, tetracycline, macrolide, fluoroquinolone, and sulfonamide.

In yet another preferred embodiment said anti-inflammatory agent is a TNF-alpha- blocker, an IL1-blocker or an IL-6 blocker or a corticosteroid or a metabolically active anti-inflammatory agent.

It is particularly preferred that said IL1-blocker is canakinumab, anakinra or rilonacept.

It is further particularly preferred that said IL6-blocker is tocilizumab, sarilumab, or siltuximab.

It is further particularly preferred that said TNF-alpha blocker is infliximab, adalimumab or etanercept.

In yet another preferred embodiment said metabolically active anti-inflammatory agent is ethyl lactate such as ethyl D-lactate (DEL) or ethyl L-lactate (LEL), propyl lactate, butyl lactate such as butyl D-lactate (DBL) or butyl L-lactate (LBL), or ethyl-2-hydroxybutanoate).

In yet another preferred embodiment, the pharmaceutical composition for use according to the invention comprises one or more auxiliary compounds such as carriers, diluents, fillers, flavouring agents, and stabilizers.

In a further embodiment, the pharmaceutical composition for use is for topical or systemic administration.

In a further embodiment, the pharmaceutical composition for use is for percutaneous, oral, intravenous, intranasal, or pulmonary administration, or for administration as an aerosol or as spray.

In yet another preferred embodiment, the pharmaceutical composition for use is to be administered via an inhaler device or a spraying device.

In a further preferred embodiment said pharmaceutical composition for use is to be administered within 48 h of the onset of at least one sign or symptom of said disease, preferably within 40 h, 36 h, 30 h, 24 h, more preferably less than 24 h of the onset of at least one sign or symptom of said disease.

In yet another preferred embodiment said pharmaceutical composition for use is to be administered for 2 days or more, preferably for 2 to 14 days.

In preferred embodiments of the compound for use, or pharmaceutical composition for use of the present invention, said disease is caused by an influenza virus.

In a further embodiment said at least one sign or symptom of influenza is one or more of fever or feeling feverish, chill, cough, sore throat, runny or stuffy nose, sneezing, muscle or body ache, headache, Fatique, vomiting, diarrhea, respiratory tract infection, chest discomfort, shortness of breath, bronchitis, and pneumonia.

In yet another embodiment said at least one sign or symptom of said virus caused disease is relieved.

In a particularly preferred embodiment, the virus is an influenza virus.

In a further particularly preferred embodiment said influenza virus is an influenza A, B, C or D virus.

In yet another particularly preferred embodiment said influenza virus is a type H1N1, H1N2, H1N3, H1N8, H2N1, H2N2, H2N3, H3N1, H3N2, H3N8, H4N1, H5N1-H5N9, H9N2, H6N1, H7N1-H7N9, H10N8, H9N2, H10N7, H10N8, H11N2, H11N9, H17N10, H12N1, H13N2, H13N9, H14Nx, H15Nx, H16N3, H17N10, or H18N11 influenza virus.

According to the invention said disease is influenza.

In a further preferred embodiment said subject as mentioned above is an immunosuppressed or immunodeficient mammal. It is particularly preferred that said immunosuppression or immunodeficiency is associated with hereditary or acquired immuno-defects.

It is particularly preferred that said acquired immune defect is associated with HIV, organ transplantation, chemotherapy or exposure to radiation.

### BRIEF DESCRIPTION OF THE FIGURES

**Figure 1** shows schematically the live cycle of a sialosugar-binding virus.

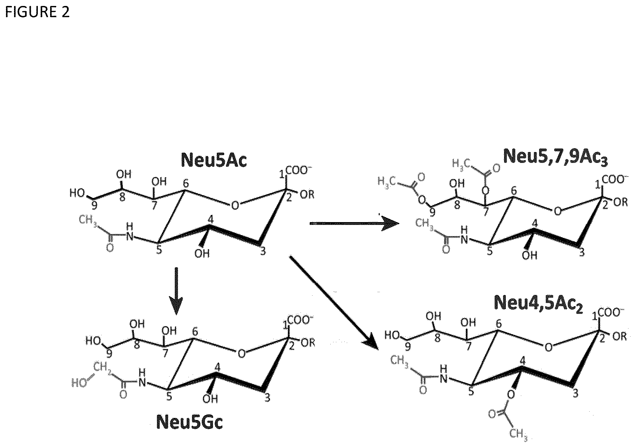

**Figure 2** shows the core structure of sialic acids and its derivatives.

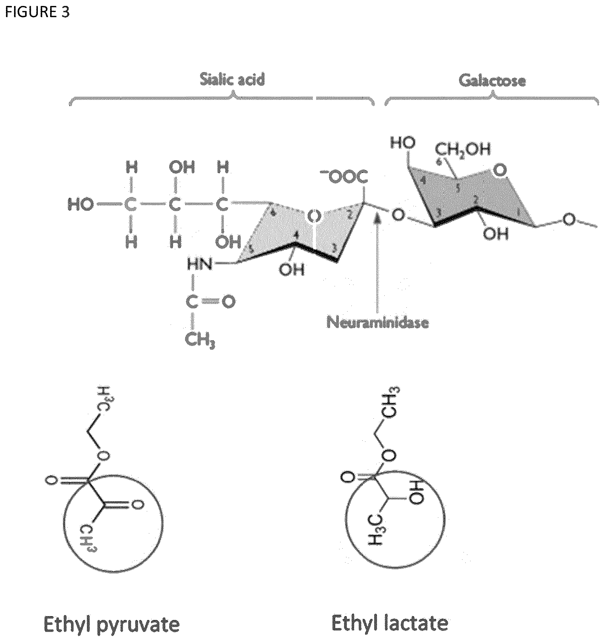

**Figure 3** shows the site of activity of neuraminidase.
**Figure 4** shows the effect of ethyl pyruvate and ethyl lactate on the activity of neuraminidase.
**Figure 5** shows the effect of ethyl pyruvate and ethyl lactate on virus-infected cells.
**Figure 6** shows the inhibitory effect of ethyl pyruvate and ethyl lactate on release of inflammatory cytokines from blood cells.
**Figure 7** shows the effect of ethyl pyruvate and ethyl lactate on bacterial growth on blood agar plates.
**Figure 8** shows the effect of ethyl pyruvate on the vitality of primary human fibroblasts.
**Figure 9** shows the differential effect of ethyl pyruvate and pyruvate on proliferation of growth-stimulated LNCaP cells.

### DETAILED DESCRIPTION OF THE INVENTION

Although the present invention will be described with respect to particular embodiments, this description is not to be construed in a limiting sense. Nevertheless, the invention is defined in the claims.

Before describing in detail exemplary embodiments of the present invention, definitions important for understanding the present invention are given.

As used in this specification and in the appended claims, the singular forms of "a" and "an" also include the respective plurals unless the context clearly dictates otherwise

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" or "essentially consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "(i)", "(ii)", "(iii)" or "(a)", "(b)", "(c)", "(d)", or "first", "second", "third" in the description or in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms relate to steps of a method or use there is no time or time interval coherence between the steps, i.e. the steps may be carried out simultaneously or there may be time intervals of seconds, minutes, hours, days, weeks. between such steps, unless otherwise indicated.

It is to be understood that the terminology used herein is for the purpose of describing particular embodiments only and is not intended to limit the scope of the present invention that will be limited only by the appended claims. Unless defined otherwise, all technical and scientific terms used herein have the same meanings as commonly understood by one of ordinary skill in the art.

As has been set out above, the present invention concerns in one aspect ethyl pyruvate for use in the treatment or prophylaxis of influenza.

The compound for use according to the present invention is ethyl pyruvate, which is also shown in the following Table 2.

**Table 2: The compound according to the invention**

| **Name** | **Formula** |
|---|---|
| Ethyl pyruvate | CH3-CH2-O-CO-CO-CH3 |

In the context of the present invention, it was found that compounds of the present invention, i.e. ethyl pyruvate are capable of acting competitively with the O- or N-linked acetyl residues of various sialic acids or sialic acid variants. The compound thus prevents viruses from binding to or docking with sialic acid-containing oligosaccharides or sugar polymers. Typical binding partners are hemagglutinins or lectin-like surface proteins of a virus, e.g., the S1 / S2 spike protein of coronaviruses. In addition, the compound also inhibits viral neuraminidases.

The present invention relates to ethyl pyruvate, its use for the preparation of medicaments, pyruvate for use in the treatment or prophylaxis of influenza.

In a specific aspect the present invention relates to a pharmaceutical composition for use comprising the compound as defined herein. The pharmaceutical composition is for use in the treatment or prophylaxis of influenza. Furthermore, the pharmaceutical composition is for use in inducing regression or elimination or inhibition of the progression of at least one sign or symptom of influenza, in a subject.

Definitions in the context of pharmaceutical compositions are to be understood as also relating to connected activities such as the manufacture of a medicament, its medical use or a method of treatment based on the usage of the medicament, compound or pharmaceutical composition.

In a central embodiment of the present invention a pharmaceutical composition for use comprises the compound for use of the invention as defined herein.

In one embodiment, the pharmaceutical composition for use of the invention may comprise the compound for use according to the invention as the sole active ingredient. This does not exclude the presence of non-pharmaceutically active additives, i.e., substances which contribute to preparing a galenic formulation, such as fillers, flavouring agents, stabilizers.

The pharmaceutical composition for use according to the invention can further comprise one or more additional pharmaceutically or therapeutically active ingredients. In the context of combinations with further active ingredients the low toxicity of the compound of the present invention is of particular advantage.

It is preferred that the additional therapeutically active ingredient is an antiviral compound, an anti-inflammatory compound, an anti-bacterial agent and/or an analgesic.

Envisaged examples of antiviral compounds are agents which generally interfere with the virus' life cycle, replication and dispersal strategy. Preferably, the compound interferes with the binding of the virus to a cellular receptor. In a further embodiment, the compound is an entry inhibitor, an uncoating inhibitor, a reverse transcriptase inhibitor, an integrase inhibitor, a protease inhibitor, a viral assembly inhibitor, pyrazinecarboxamide, a neuraminidase inhibitor, an influenza polymerase domain inhibitor, an influenza CAP-binding PB2 domain inhibitor or a viral release inhibitor. Preferred examples of antiviral compounds which may be used together with the compound(s) according to the present invention include one or more of the group of: Abacavir; Acyclovir; Adefovir; Amantadine; Ampligen; Amprenavir; Arbidol; Atazanavir; Atripla; Balavir; Baloxavir marboxil; Biktarvy; Boceprevir; Cidofovir; Cobicistat; Combivir; Daclatasvir; Darunavir; Delavirdine; Descovy; Didanosine; Docosanol; Dolutegravir; Doravirine; Ecoliever; Edoxudine; Efavirenz; Elvitegravir; Emtricitabine; Enfuvirtide; Entecavir; Etravirine; Famciclovir; Fomivirsen; Fosamprenavir; Foscarnet; Fosfonet; Fusion inhibitor; Ganciclovir; Ibacitabine; Ibalizumab; Idoxuridine; Imiquimod; Imunovir; Indinavir; Inosine; Integrase inhibitor; Interferon type I; Interferon type II; Interferon type III; Lamivudine; Letermovir; Lopinavir; Loviride; Maraviroc; Methisazone; Moroxydine; Nelfinavir; Nevirapine; Nexavir; Nitazoxanide; Norvir; Nucleoside analogues; Oseltamivir; Peginterferon alfa-2a; Peginterferon alfa-2b; Penciclovir; Peramivir; Pleconaril; Podophyllotoxin; Pyramidine; Raltegravir; Remdesivir; Reverse transcriptase inhibitor; Ribavirin; Rilpivirine; Rimantadine; Ritonavir; Saquinavir; Simeprevir; Sofosbuvir; Stavudine; Synergistic enhancer; Telaprevir; Telbivudine; Tenofovir alafenamide; Tenofovir disoproxil; Tenofovir; Tipranavir; Trifluridine; Trizivir; Tromantadine; Truvada; Valaciclovir; Valganciclovir; Vicriviroc; Vidarabine; Viramidine; Zalcitabine; Zanamivir; Zidovudine, and an antiviral plant extract. The term "antiviral plant extract" as used herein relates to mixture plant extracts of plants which are known for their antiviral activity. The group of plants includes 2, 3, 4, 5, 6, 7, 8, 9, 10, 11, 12, 13, 14, 15, 16, 17, 18, 19, 20, or more or all of the following plants or plant extracts: Heracleum maximum, Plantago major Linn, P. asiatica Linn, Trifollium species, Pandanus, Amaryllifolius Roxb, Carissa edulis, Phyllanthus urinaria L., Astzragalus chinensis, Geranium sanguineum L., Elderberry extract, Boehmeria nivea L., Saxifraga melanocentra. Olea europaea L., Herba Echinacea, Phyllanthus amarus, Azadirachta indica Juss., Rhizomata Glycyrrhizae, Artemisia Annua, Aloe vera, Withania somnifera, Uncaria tomentosa, Zingiber officinale Roscoe, Gingko biloba, Eleutherococcus senticosus, Olive leaf extract.

A suitable and envisaged example of an anti-inflammatory compound is ibuprofen, naproxen, diclofenac, diflunisal, etodolac, flurbiprofen, indomethacin, ketoprofen, mefenamic acid, meloxicam, nabumetone, oxaprozin, piroxicam or sulindac.

Further envisaged anti-inflammatory agents are anti-cytokine agents, preferably TNF-alpha-blocker, IL1-blocker, IL-6 blocker; or a corticosteroids or metabolically active anti-inflammatory agents.

A suitable and envisaged example of an IL1-blocker is canakinumab, anakinra or rilonacept.

A suitable and envisaged example of an IL6-blocker is tocilizumab, sarilumab, siltuximab.

A suitable and envisaged example of an TNF-alpha blocker is infliximab, adalimumab or etanercept.

A suitable and envisaged example of a metabolically active anti-inflammatory agent is ethyl lactate (EL) such as ethyl D-lactate (DEL) or ethyl L-lactate (LEL), propyl lactate, butyl lactate such as butyl D-lactate (DBL) or butyl L-lactate (LBL), or ethyl-2-hydroxybutanoate).

Suitable and envisaged examples of antibacterial agents include antibiotics comprising one or more selected from beta-lactam antibiotics (penicillins, ampicillins, carbenicillins, methicillin, ticarcillin, cephalosporin, imipenem, aztreonam), azithromycin, aminoglycosides (gentamycin, kanamycin, neomycin, netilmicin, streptomycin, tobramycin), tetracyclines (demeclocyclin, doxycyclin, minocyclin, oxytetracyclin), macrolide (azithromycin, clarithromycin, clindamycin, erythromycin, lincomycin), quinolones and fluoroquinolones (cinoxacin, nalidixic acid, ciprofloxacin, enoxacin, norfloxacin, levofloxacin, lomefloxacin), sulfonamides (sulfisoxazole, sulfamethoxazole, sulfadiazine, sulfacetamide), oxazolidones (linezolid), biocidal peptides (polymyxin, polymyxin B, colistin, bacitracin, like defensins), trimethoprim, trimethoprim-sulfamethoxazole, cloramphenicol, vancomycin, metronidazol rifampicin, rifampin, doxocyclin, gramicidin, cycloserin and isoniazid. Particularly preferred are penicillins, aminoglycosides, tetracyclines, macrolides, fluoroquinolones, and sulfonamides.

A suitable and envisaged example of an analgesic is a selective COX-2 inhibitor, an opioid, or an NSAID. The present invention in particular envisages the employment of acetaminophen, ibuprofen, naproxene, hydrocodone, oxycodone, diclofenac, piroxicam, etodolac, fenoprofen, ketoprofen, ketorolac, meclofenamate, meloxicam, nabumetone, a cyclooxygenase-inhibitor, or tramadol.

The prevent invention further envisages the combination of ethyl pyruvate with oxamate, an inhibitor of lactate dehydrogenase. Also envisaged is the combination of the compounds of the present invention and an inhibitor of the glycerol aldehyde phosphate dehydrogenase, such as iodide acetate.

The pharmaceutical composition for use according to the present invention may comprise the compound according to the present invention in combination with one or more additional agents, e.g., agents fulfilling different functions such as antibiotics, or different agents fulfilling the same function such as two or more different antibiotics.

In a further specific embodiment, the present invention relates to the use of compound of the present invention in combination with known or novel genetic methods like siRNA and antisense nucleotides for the targeted inhibition of enzymes or proteins.

The pharmaceutical composition for use or medicament can further comprise one or more auxiliary substances useful for the galenic formulations of drugs, including, but not limited to, fillers, flavouring agents, stabilizers and agents that prevent microbial growth in the pharmaceutical composition, propellants for inhalation, sequestering agents for stabilization of inhalation compositions, solvents or co-solvents.

The pharmaceutical composition for use can be provided or used in any suitable galenic formulation, depending on the disease it is used against, e.g., a specific virus infection to be treated, and/or the chosen route of administration. The skilled person can readily select and prepare a suitable preparation on the basis of common general knowledge. Pharmaceutical compositions for use of the invention can be prepared according to known methods e.g., by mixing one or more effective substances with one or more carriers, and forming of e.g., aerosols or aerosol formulations, tablets, capsules, or liquid solutions. Where appropriate, solutions can, for example, be encapsulated in liposomes, microcapsules or other forms of containment.

Examples of suitable formulations comprise aqueous solutions which can optionally be buffered, water in oil emulsions, oil in water emulsions, creams, ointments and formulations comprising any of the foregoing.

The invention, in further embodiments, envisages a pharmaceutical composition for use prepared in the form of a sustained release or controlled release galenic formulation. Such formulations allow the targeted release in e.g., a certain location, such as a certain part of the stomach or intestine, or a certain tissue or organ, and/or allow the sustained release over a defined period of time.

In specific embodiments, the pharmaceutical composition for use according to the present invention may be provided in the form of nasal drops, a spray, or an inhalant (droplet) form. Thereby it is possible to provide the pharmaceutical composition for use according to the present invention directly to the affected area. It can be maintained at a high concentration. Specifically, regarding nasal drops, spray, and inhalant (droplets), the pharmaceutical composition for use according to the present invention may previously have been administered to, for example, the nasal cavity and the pharyngeal mucosa, and the lung. The pharmaceutical composition for use according to the present invention may accordingly be maintained at sufficiently high concentration around the cells of the pharyngeal mucosa and the lung.

Providing a pharmaceutical composition for use according to the present invention having the form of ointment or lotion makes it further possible to maintain the pharmaceutical composition for use at a sufficiently high concentration in the affected area.

It is particularly preferred to provide the pharmaceutical composition for use according to the present invention in the form of a composition suitable for inhalation or direct use in the respiratory tract of a subject, e.g., in the mouth, pharynx and lungs of a subject. Accordingly, the present invention envisages the formulation of the pharmaceutical composition for use as composition suitable for aerosol administration. It is particularly envisaged to use the pharmaceutical composition for use with pressurized metered dose inhalers (MDIs) suitable for aerosol administration. These MDI may use a propellant to expel droplets containing the pharmaceutical composition for use to the respiratory tract as an aerosol. Suitable examples of such propellants include, for example, hydrofluoroalkanes (HFAs) such as 1,1,1,2-tetrafluoroethane (HFA 134a) or 1,1,1,2,3,3,3-heptafluoropropane (HFA 227) which have been acknowledged to be the best candidates for non-CFC propellants. A number of medicinal aerosol formulations using such HFA propellant systems have been disclosed. Particularly preferred is the use of HFA 134a, HFA 227 and mixtures thereof.

The pharmaceutical compositions for use to be used for inhalation according to the present invention, may further contain excipients and in particular a low volatility component in order to increase the mass median aerodynamic diameter (MMAD) of the aerosol particles on actuation of the inhaler.

The pharmaceutical composition for use of the invention comprises at least one compound of the invention in a therapeutically effective concentration or amount. The skilled person can readily determine the therapeutically effective concentration or amount in standard in vitro or in vivo experiments. For example, the effective amount can be estimated based on an extrapolation from in vitro data, such as enzyme inhibition or cellular assays.

For example a dosage can be formulated in animal models which corresponds to the IC50 in cell culture experiments. Hence, according to commonly known methods, the optimal dosage for a vertebrate to be treated, such as a human, can be deduced from animal experiments. The amount of the agent to be administered naturally depends on the person to be treated, his body weight, his genetic and physical constitution, the disease state, the route of administration, the galenic formulation and other parameters.

Furthermore, dosage and the interval of administration may depend on the respective clinical situation of the patient to guarantee a therapeutic effect. Generally, useful effective concentrations, i.e., concentrations to be achieved at the level of cellular exposure, may range from at least 0.05 mM up to 500 mM, e.g., including 1, 2, 3, 4, 5, 10, 10, 20, 30, 40, 50, 60, 70, 80, 90, 100, 150, 200, 250, 300, 400, 450, 500mM or any value in between the mentioned values. The concentration typically depends on the route of administration, the tissue type and other factors and can accordingly be adapted or modified in line with the skilled person's common general knowledge.

According to particularly preferred embodiments, the therapeutically effective concentration or amount of a pharmaceutical composition for use or of a compound for use according to the present invention may be specifically adapted to the preferred route of administration, e.g., inhalation. As is known in the art, the amount or concentration or active ingredient used for inhalation is higher than the amount to be used for other routes of administration. It is accordingly preferred to use a suitably defined concentration. For example, to achieve a therapeutic effect of the pharmaceutical composition for use when applied by inhalation the optimal therapeutic concentration must be tested. Useful effective concentrations, i.e., concentrations to be achieved at the site of cellular exposure, range from at least 5 mM, preferably from 5 mM to 50 mM, more preferably from 5 to 200 mM, most preferably from 5 mM to 500 mM in the context of inhalation.

In case of systemic application, the effective concentrations to be applied may be different, preferably 1 mM to 20 mM, most preferably 1 mM to 100 mM, e.g., when given by infusion. In topical applications higher concentrations may be useful. Preferred are 0.2 to 200 mM, more preferred are 0.2 to 50 mM and 50 to 200 mM.

In other words, the concentrations above refer to desired blood and/or tissue concentrations, or local concentrations. Thus, the invention relates to pharmaceutical preparations for use suitable to achieve such concentrations upon administration.

To achieve a therapeutic effect the pharmaceutical composition for use of the present invention is generally applied for 1, 2, 3 or more days or weeks as repeated bolus doses (e.g. injections, or inhalation) or continuous administration (e.g. infusion), or any time period required to achieve a therapeutic effect, at the respective therapeutically effective dosage. Because of their nature, esters are of limited stability, which may require the use of higher and/or repeated doses for all kind of applications. In preferred embodiments, the pharmaceutical composition for use is to be administered for 2 days or more. More preferably, the pharmaceutical composition for use is to be administered for 2 to 14 days.

Furthermore, dosage and the interval of administration can be guided by the individual plasma concentrations of the agent that guarantee a therapeutic effect.

The pharmaceutical composition for use of the present invention can, in further embodiments, be administered topically or systemically. In both topical and systemic administrations, a local administration to a selected site can be performed.

Pharmaceutical compositions for use comprising the compounds of the invention can be administered according to any suitable or generally known method - including percutaneous, oral, intravenous, or pulmonary administration, or administration as an aerosol, nasal drops, via mini-pumps, as mouth lavage, gel, ointment, cream, spray, oil, lipstick, plaster, via microbubbles and/or by inhalation or via an inhalator. It is particularly preferred to administer the pharmaceutical composition for use via inhalation as outlined above. Further preferred is to administer the pharmaceutical composition for use as spray or via a spraying device, e.g., as aerosol. Further details would be known to the skilled person or can be derived from suitable literature sources such as Anthony J. Hickey, Sandro R. da Rocha, Pharmaceutical Inhalation Aerosol Technology, CRC Press, Third Edition, 2019.

In particularly preferred embodiments, the pharmaceutical composition for use is to be administered via the respiratory tract and/or the mouth. It is further particularly preferred to administer the pharmaceutical composition for use via an inhaler device or a spraying device.

Administration may, in further embodiments, be for example systemic, e.g., by single or repeated oral or parenteral application, or via methods wherein the medicament is to be administered systemically in an inert vehicle and is only released at the desired location by respective manipulation. An example thereof is, amongst others, so called microbubbles.

The pharmaceutical composition for use can also be a provided as food supplement or beverage supplement. In the context of the present invention, "food supplement" or "beverage supplement" means a pharmaceutical composition for use that is administered together with the standard daily diet, or a special medical diet. It also means "health food", i.e. food of a particular composition that is consumed by subjects without medical supervision to achieve a prophylactic or therapeutic effect. In specific embodiments, the pharmaceutical composition for use is formulated as drink or beverage, i.e., is mixed with water or other beverages and may then be orally administered.

A subject to be treated may be a mammal, in particular a human being. In certain embodiments the mammals may be dogs, cats, swine, cattle, monkeys, goats, sheep, mice, rats and others. Also envisaged are other mammals. In certain embodiments also non-mammalian animals may be treated, e.g., chicken or other types of birds. It is further preferred that the non-mammalian animal is an avian species. Envisaged examples of avian species include poultry such as chicken, turkeys, ducks, geese, guinea fowls or pigeons.

The term "treatment and/or prophylaxis of a disease associated with an interaction of a receptor comprising a sialic acid-containing sugar chain with a cognate binding element" typically encompasses virostatic effects. Without wishing to be bound by theory, the treatment approach is assumed to be based on a competitive binding of the compound of the present invention to various sialic acids or sialic acid variants. The compound thereby prevents binding elements such as those present on a virus, from binding to or docking with sialic acid-containing oligosaccharides or sugar polymers. Additional, secondary effects may be observed when the compound of the present invention enters the affected or diseased cell. Since a virus during a viral infection typically makes use of at least part of the cellular machinery and also the cellular energy resources during its replication and multiplication, the cellular machinery is misused for viral activities which typically involves a high energy turnover. The virus accordingly depends on energy gained by cellular mechanisms such as oxidizing glycerolaldehyde phosphate to pyruvate (glycolysis). Since mammalian cells such as epithelial cells or avian cells utilize glycolysis, as well as detoxifying glyoxalases, it is suggested that the inhibition of glycolytic enzymes by compounds according to the present invention, i.e. ethyl pyruvate, induce cell death or apoptosis and thereby reduce and ultimately eliminate the viral infection process. At the same time the compound according to the present invention is also capable of reducing, for example, bacterial or fungal infections, since bacteria and fungi also make use of glycolysis and thus are vulnerable to the inhibition of glycolytic enzymes by compounds as defined above. This allows for a unique treatment of not only virus infections, but also additional bacterial and/or fungal infections, which may be present as additional complication during a virus infection. The pharmaceutical compositions for use or compounds for use of the present invention thus have no direct virusdestroying effect, but indirectly and significantly or completely abolish virus entry into cells and reduce or eliminate the viral replication in cells or tissues which were treated.

The term "virus infection" as used herein encompasses viral infection of certain tissues, e.g., of the respiratory tract, the brain, the gastrointestinal tract, the liver or the mucosa as well as systemic infections, including infections of general tissues and organs. Compounds of the invention are used for the treatment of mucosal (topical), respiratory tract or lung diseases and/or systemic diseases.

The term "bacterial infection" encompasses superficial colonisation by bacteria, e.g., of the skin, intraauricular, respiratory tract or mucosa as well as systemic infections, including infections of blood, tissues and organs. Also encompassed are infections of the gastrointestinal tract. Compounds of the invention may be used are used for the treatment of mucosal (topical) and/or systemic diseases. The mucosal diseases can be caused by oral or vaginal infections. The oral or vaginal infections are for example the consequence of AIDS, chemotherapy or an immune suppressive therapy or immune suppressive conditions.

The term "treatment" encompasses the provision of pharmaceutical compositions for use as defined herein to subjects suffering from a disease as defined herein, preferably virus infection stages or stages of virus and bacterial/fungal infection, such as acute or chronic infection, and may also encompass after-treatment as well as prophylaxis. "After-treatment" means a treatment following conventional therapy. Treatment concomitantly with conventional therapy (e.g., further antiviral treatment or treatment with antibiotics) is also part of the present invention.

The term "prophylaxis" as used herein relates to the administration of a pharmaceutical composition for use of the invention when a subject is at risk to develop a disease as defined herein, preferably a virus infection or a combination of a virus and bacterial/fungal infection, or a disease/infection is suspected or is present sub-clinically, but said disease/ nfection has not fully evolved or has not been diagnosed.

The present invention further envisages the elimination or inhibition of the progression of at least one sign or symptom of said disease. The "sign or symptom of the disease" is understood as a typical sign or symptom associated with an influenza virus infection. Such signs or symptoms include one or more of fever or feeling feverish, chill, cough, sore throat, runny or stuffy nose, sneezing, muscle or body ache, headache, Fatique, vomiting, diarrhea, respiratory tract infection, chest discomfort, shortness of breath, bronchitis, and pneumonia. A subject may develop only one, two or three of these symptoms, or more than three, e.g. all or all but one, two or three or more. The administration of a pharmaceutical composition for use comprising a compound according to the present invention relieves one, two, three or more, preferably all of the mentioned signs or symptoms.

According to preferred embodiments a pharmaceutical composition for use according to the present invention may be administered to a subject, e.g. a subject in need thereof, within 48 h of the onset of at least one sign or symptom as mentioned above. More preferably a pharmaceutical composition for use according to the present invention may be administered to a subject, e.g., a subject in need thereof, within 40 h, 36 h, 30 h, 24 h of the onset of at least one sign or symptom as mentioned above. Even more preferably a pharmaceutical composition for use according to the present invention may be administered to a subject, e.g. a subject in need thereof, less than 24 h of the onset of at least one sign or symptom of said disease as mentioned above, e.g. within 1, 2, 3, 4, 5, 6,7 ,8 , 8, 10, 11, 12, 13, 14, 15, 16, 17, 18, 19, 20, 21, 22, 23 h of the onset of at least one sign or symptom of said disease as mentioned above. The treatment duration, e.g., of an inhalation treatment, may have any suitable length and frequently per day. It is preferred that the duration is 1 to 20 min, e.g. 1, 2, 3, 4, 5, 6, 7, 8, 9, 10, 11, 12, 13, 14, 15, 16, 17, 18, 19, 20 min or more per day. The frequency may be 1, 2, 3, 4, 5 or more times per day, preferably 1 time per day.

According to the invention said disease is influenza.

In further embodiments, said virus infection may be provoked or induced by an influenza virus. Preferably, the virus is an influenza A, B, C or D virus. Even more preferably the influenza virus is a type H1N1, H1N2, H1N3, H1N8, H2N1, H2N2, H2N3, H3N1, H3N2, H3N8, H4N1, H5N1-H5N9, H9N2, H6N1, H7N1-H7N9, H10N8, H9N2, H10N7, H10N8, H11N2, H11N9, H17N10, H12N1, H13N2, H13N9, H14Nx, H15Nx, H16N3, H17N10, or H18N11 influenza virus.

By making use of pharmaceutical compositions for use and compounds use according to the present invention, several of viral life cycle steps, in particular those which require the consumption of energy in the form of ATP can advantageously be stopped, thus abolishing the viral replication within the cell.

In a further embodiment, the present invention relates to the treatment or prophylaxis of a disease, e.g., a virus infection in a mammal or avian species, concomitant with a bacterial infection. As outlined above, the compound for use according to the present invention is not only capable of competitively preventing the viral infection and reducing or eliminating the viral replication in a mammalian or avian cell, it also is capable of killing bacterial cells. Accordingly, influenza may be treated together with a bacterial infection by a pathogenic bacterium. In a specific embodiment, the concomitant bacterial infection may be caused by aerobic and/or anaerobic bacteria, or gram positive and/or gram negative bacteria. In further specific embodiments, the bacterial infection may be caused by the bacterium belonging to the genus of Actinobacillus, Actinomyces, Bacteroides, Clostridium, Campylobacter, Enterobacter, Enterococcus, Eubacterium, Fusobacterium, Helicobacter, Peptostreptococcus, Pneumococcus, Porphyromonas, Prevotella, Pseudomonas, Spirochetes, Staphylococcus, Streptococcus, or Treponema.

In further specific embodiments, the concomitant bacterial infection to be treated or prevented may be caused by one or more bacteria belonging to the genus Acrobacter, Borrelia, Bacillus, Brucella, Clamydia, Corynebacterium, Cryptococcus, Erythrobacter, gram-positiv cocci, Hemophilus, Legionella, Listeria, Mycobacteria, Mycoplasma, Neissaria, Pasteurella, Rickettsia, Salmonella, Vibrio, Yersinia, more specifically, Escherichia coli, Pneumocystis carinii, Helicobacter pylori or Borrelia burgdorferi.

In a further embodiment, the concomitant bacterial infection to be treated may be caused one or more bacteria of the group of Porphyromonas gingivalis, Prevotella intermedia, Actinomyces, Eubacterium nodatum, Peptostreptococcus micros, Peptostreptococcus anaerobius, Campylobacter rectur, Neisseria, Treponema denticola, Tannerella forsynthensis, Staphylococus aureus and Fusobacterium nucleatum.

Accordingly, the term "treating a bacterial infection" as used herein accordingly relates to the treatment of a clinically manifest bacterial infection associated disease. It is meant to encompass both cytotoxic and cytostatic effects on bacterial cells, thus leading to an inhibition of bacterial cells, which typically encompasses the inhibition of cell proliferation (bacteristatic action) as well as the killing of the cells (bactericidal action). The killing of bacterial cells by necrosis or apoptosis is further encompassed by the invention. The terms "proliferation" and "growth" are used interchangeably in the context of this application.

In further embodiments of the invention, the concomitant bacterial infection to be treated or prevented may be caused by a bacterium or bacterial strain that is resistant to conventional antibacterial agents. For example, the bacterium or bacterial strain may be resistant to one or more selected from the group comprising penicillins, aminoglycosides, tetracyclines, macrolide, fluoroquinolones, sulfonamides, vancomycin, trimethoprim, ciprofloxacin, oxazolidinones, linezolid, isoniazid, rifampin, methicillins and/or exhibit resistance due to expression of beta-lactamase, antibiotics transporter molecules, RNA methylation, and/or that resistance is due to other genetic changes of the bacteria.

In particularly preferred embodiments, said concomitant bacterial infection may be due to a bacterium which is resistant to beta-lactamase antibiotics and/or to methicillin.

In further particularly preferred embodiments, said concomitant bacterial infection to be treated according to the present invention may be due to an MRSA or EHEC infection. Advantageously, the compounds of the present invention are capable of also inhibiting bacteria such as methicillin-resistant staphylococcus aureus (MRSA) or EHEC, which pose severe problems in the clinical setting, in particular in combination with virus infections as mentioned above.

In yet another set of embodiments, the virus infection to be treated or prevented may be present or may potentially develop in a mammal or avian species concomitant with a fungal infection. In yet another embodiment, the fungal infection may further be present or may potentially develop together with a bacterial infection as described above.

A fungal infection as mentioned herein, may, for example, be caused by one or more selected from group comprising Candida spp., Aspergillus spp., Cryptococcus spp., Pneumocystis spp., Zygomyces spp., Dermatophytes, Blastomyces spp., Histoplasma spp., Coccidoides spp., Sporothrix spp. Microsporidia spp., Malassezia spp. and Basidiomycetes. Some of the corresponding infectious diseases caused by said fungi may be an opportunistic infection, and/or may be characterized by antibiotic resistance.

Viral and bacterial and/or fungal infections further represent a significant problem in patients in an immunosuppressed state, and are amongst the leading cause of death, e.g. in transplant patients. Accordingly, in a further preferred embodiment, the pharmaceutical composition for use according to the present invention is for use in an immunosuppressed or immunodeficient mammal or avian species. Said immunosuppression or immunodeficiency is associated with hereditary or acquired immuno-defects. For example, said acquired immune defect is associated with HIV, organ transplantation, chemotherapy or exposure to radiation.

The following examples and figures are provided for illustrative purposes.

### EXAMPLES

### Example 1

### Life cycle of a sialosugar binding virus

Sialosugar binding viruses like the influenza virus attach to host cells via interaction of hemagglutinin with surface receptors containing sialic acid residues in the glycoconjugate parts. After internalization and uncoating of the of the virus, its genetic material, RNA, is release into the cell and replicated by the RNA polymerase. New viral RNA and viral proteins synthesized by the host cell assemble at the cell surface into new virions. After budding, virions need to be detached from the host cell by viral neuraminidase which release the virion by splitting of sialic acid from the cell surface. Indicated are agents that interfere with virus binding to receptors and virus release after budding.

**Figure 1** shows how competitive drugs according to the invention interfere with the viral replication cycle.

### Example 2

### Structure of sialic acid and its derivatives

The core sialic acid structure contains nine carbons, and modifications are commonly found at positions 4, 5, 7, 8 and 9. Modifications at the 5-carbon position determine the primary sialic acid forms: N-acetylneuraminic acid (Neu5Ac), and N-glycol-ylneuraminic acid (Neu5Gc). Approximately 20 different sialyltransferases attach sialic acid to glycans via α2-3, α2-6, or α2-8 linkages.

### Example 3

### Site of activity of neuraminidase

In e.g., influenza virus, haemagglutinin as well as neuraminidase are involved in the recognition and modulation of sialic acids on the cell surface as the virus receptor. Viral neuraminidase cleaves terminal sialic acids residues from glycan structures on the surface of the infected cell. This promotes the release of progeny viruses and the spread of the virus from the host cell to uninfected surrounding cells. Neuraminidase also cleaves sialic acid residues from viral proteins, preventing aggregation of viruses. The arrow indicates the cleavage site, the circle indicates the acetyl residue at the carbohydrate backbone as well as at ethyl pyruvate.

### Example 4

### Effect of Ethyl pyruvate and ethyl lactate on activity of neuraminidase (sialidase

Neuraminidase (EC 3.2.1.18) also known as sialidase is an enzyme that hydrolyzes terminal sialic acid residues on polysaccharide chains. It is predominantly expressed on the surface of viruses and bacteria. For activity measurement, the activity was measured using the neuraminidase activity kit from Sigma Aldrich (Cat.No.: MAK121). Accordingly, the neuraminidase activity is determined by an enzymatic essay in which the sialic acid released by neuraminidase results in the colorimetric product directly proportional to neuraminidase activity in the sample. The reaction master mixture contained assay buffer, (+/-) substrate, cofactors, neuraminidase, and dye-reagent. The activity is expressed as units/L and maximum activity was arbitrarily set 100%. The effect of ethyl pyruvate and ethyl lactate on enzyme activity was evaluated by comparing the change of the absorbance against the samples with substrate (maximum activity) and without the substrate (blank).

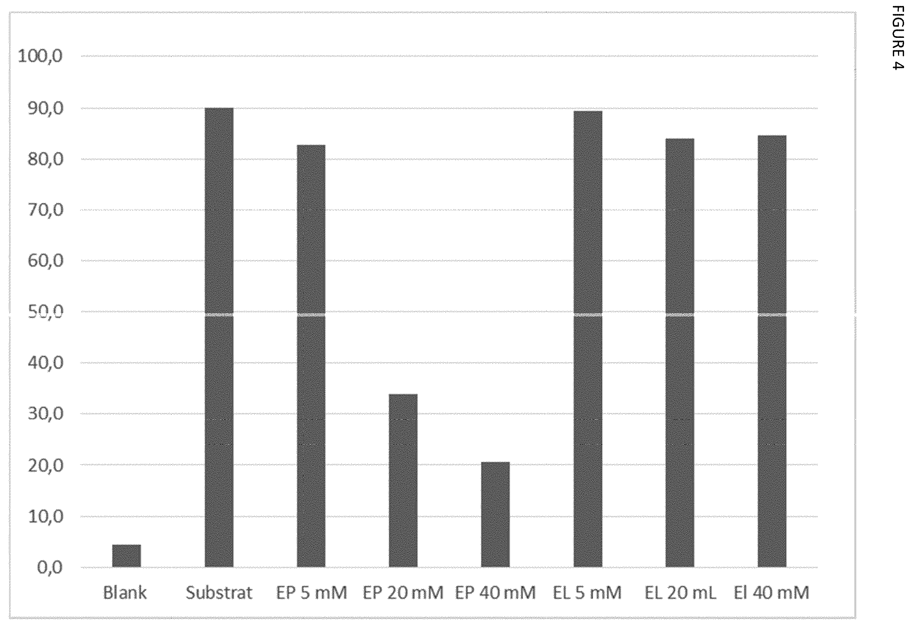

**Figure 4** shows that ethyl pyruvate inhibits activity of neuraminidase in a concentration-dependent manner while ethyl lactate exerts only minimum inhibitory effect. The data were expressed as mean of three independent experiments.

### Example 5

### Effect of Ethyl pyruvate and ethyl lactate on virus infected cells

The Madin-Darby canine kidney cells (MDCK II) (ECACC catalogue no. 85011435) were maintained in Dulbecco's modified Eagle medium (DMEM; Pan Biotech) supplemented with 10% fetal calf serum, 100 U/mL of penicillin, and 100 µg/mL of streptomycin. All cells were cultured at 37 °C in 5% CO2 and passaged twice per week, before they had reached full confluency. Cells were propagated in growth medium in 25, 75 or 175 cm2 culture flasks. Detachment of cells was achieved using trypsin-EDTA at 37 °C for 5-15 min. Trypsinization was stopped by adding growth medium. Depending on the size of the flask, around 1x105 to 1x106 cells were seeded into a new culture flask. In preparation for infection, transfection or other cell assays, cells were seeded one night prior to the experiment into 6-12-well plates or petri dishes. MDCK II cells were inoculated with the influenza virus H9N2 in medium containing 0,2µg/ml TPCK-trypsin, 25mM HEPES buffer and ethyl pyruvate and ethyl lactate at respective final concentrations. For infection, the medium was left for 1h on the cells. After incubation, the medium was removed, cells were washed with culture medium and prepared for continuous culture for 2 days. The confluency and cellular structures were analysed using a light microscope.

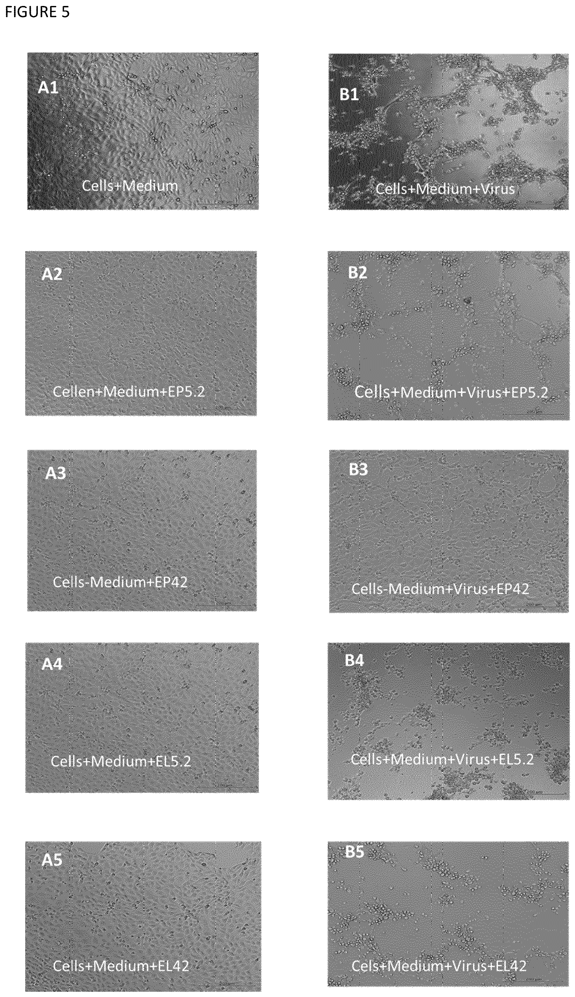

**Figure 5** shows the cytopathic effect of the influenza virus as seen in B1 characterized by loss of cell confluency, appearance of dead cells and conglomerates of cells. In contrast, in the presence of ethyl pyruvate (42 mM) the cellular structures and confluency is preserved (B3). As seen, ethyl pyruvate has no toxic effect on MDCK II cells in the absence of virus (A1-A3). In contrast, ethyl lactate does not protect MDCK II cells from the cytopathic effect of the virus (B5). Like ethyl pyruvate, ethyl lactate exhibits no toxic effect on MDCK II cells.

### Example 6

### Inhibition of release of inflammatory cytokines from human blood cells by ethyl pyruvate (EP) and ethyl lactate (EL)

Human heparinized whole blood was diluted with RPMI 1640 medium (ThermoFisher Scientific) (1:5) and incubated with LPS (10ng/ml) and increasing concentration of ethyl pyruvate (A-D) or ethyl lactate (E-H) (1 to 20 mM) for 6 h at 37°C with 5% CO₂. Following incubation, the plates were centrifuged at 2000g for 10 min and the proinflammatory cytokines were analysed by cytometric bead array using a FACSCalibur and the CellQuestTMSoftware (BD Biosciences).

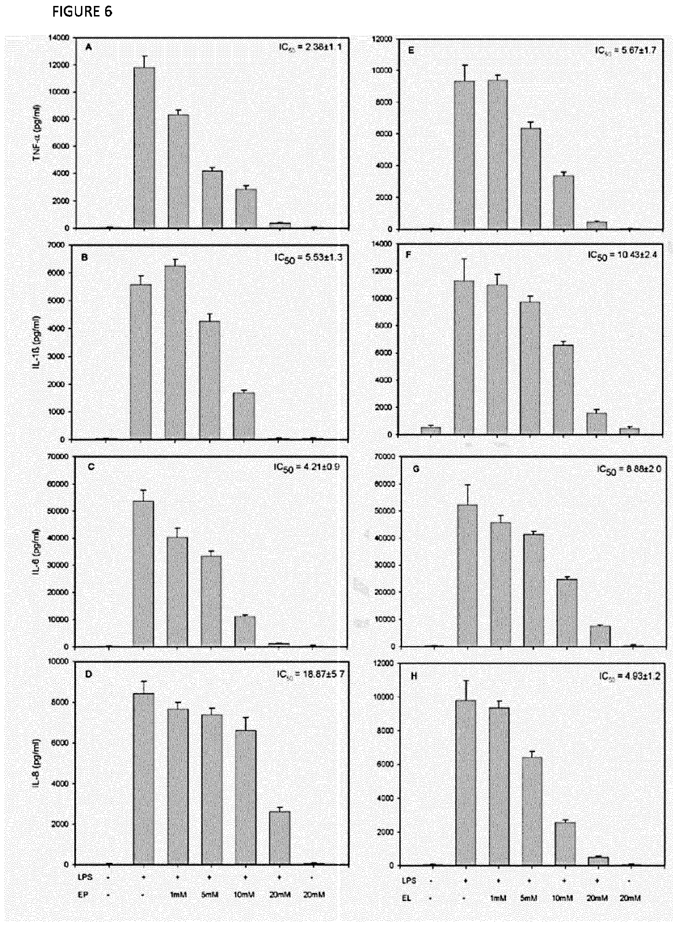

**Figure 6** shows that lipopolysaccharide (LPS)-stimulated blood cells causes the release inflammatory cytokines such as TNF-alpha (A,E), IL-1β (B,F), IL-6 (C,G) and IL-8 (D,H). The release of these cytokines can be effectively inhibited by ethyl pyruvate and ethyl lactate. Here, ethyl lactate acts as a prodrug that is enzymatically converted to ethyl pyruvate by cell enzymes.

### Example 7

### Fingerprints on blood agar plates

Upon playing in the garden by digging in the ground non-disinfected hands of a child were contacted with sterile blood agar plates containing ethyl pyruvate (middle) ethyl lactate (right) and medium (left) for 30 seconds and incubated for 48h at 37°C. The microbes displayed at the circumferences of the agar plates depict a selection of microbes tested to be sensitive against ethyl pyruvate.

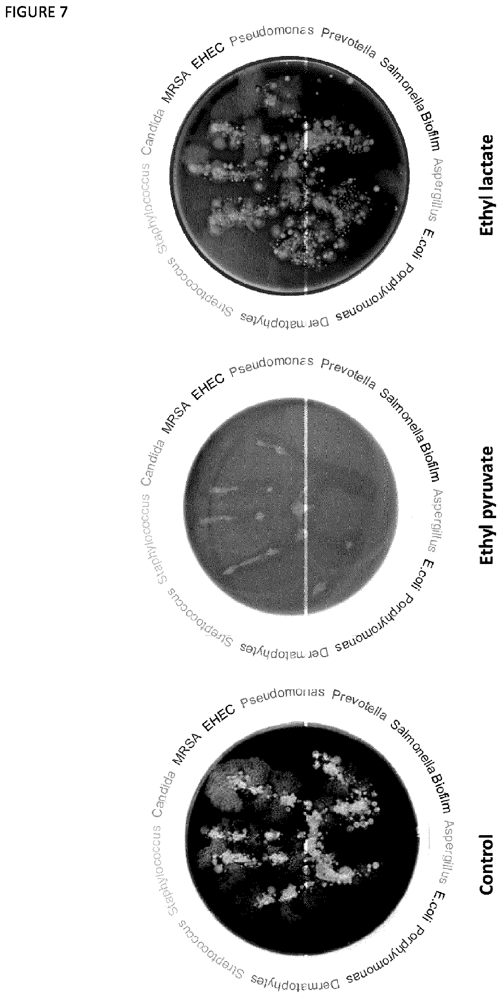

**Figure 7** demonstrates the extraordinary capacity of ethyl pyruvate in killing most pathogenic microbes. Contrarily, ethyl lactate that differs only by two protons is not effective compared to its oxidized counterpart.

### Example 8

### Effect of ethyl pyruvate (EP) on the vitality of primary human fibroblasts

An identical number (10⁴ cells per well) of primary human skin fibroblasts were inoculated in 24-well plates (Greiner, no. 662160) and were cultured in DMEM (Gibco; no. 41966-092) in the presence of 10 % calf serum (Biochrom, S0113/5), 2 mM L-glutamine (Gibco; no. 25030-024), penicillin/streptomycin (100 units penicillin/ml; 100 µg streptomycin/ml, Gibco; no. 15140/122), 5 mg% ascorbic acid (Serva, no. 14030.02) at 37°C, 5%CO2 and 95% humidity. After reaching 50 % confluency cells were 3 times washed with PBS and identical volume of fresh serum-free medium was added containing: EP (0 mM); EP (5 mM, EP (10 mM), EP (20 mM), EP (50 mM).

The culture was continued at 37°C, 5% CO2 and 95% humidity for 24 hours. Thereafter the supernatants were removed and 100 µl of a 50 % thymol blue solution was added to the wells. After washing the cells with medium the unstained and stained cells were counted under the light optical microscope comprising a coordinate plane. Cells, stained blue were assessed as avital, unstained cells as vital. The percentage of unstained cells of the total number of cells corresponds to the vitality of the cells.

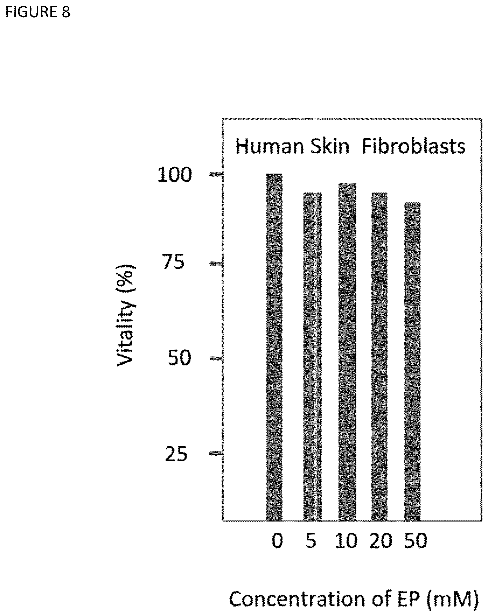

**Figure 8** shows that ethyl pyruvate (EP) is not toxic over the concentration range investigated and that it does not significantly influence vitality of primary human fibroblasts.

### Example 9

### Effect of ethyl pyruvate and pyruvate on growth of LNCaP cells growth-stimulated by androgens

An identical number of LNCaP cells (androgen dependent prostate carcinoma cells; DSMZ No ACC 256) were routinely cultured in 75 cm2 culture flasks in RPMI-1640 medium (Gibco; Nr. 21875-034), penicillin/streptomycin (100 units penicillin/ml; 100 µg streptomycin/ml; Gibco; Nr. 15140/122) in the presence of 10% fetal calf serum (Biochrom; Nr. S0113/5; RPMI-FKS). The flasks were incubated at 37°C in a humidified atmosphere (relative humidity >95%) of 5% CO2 in air. After reaching 50% confluency the medium was removed and the adherent cells were washed twice with PBS (phosphate buffered sodium chloride; 50 mM sodium phosphate, 150 mM NaCl, pH 7,4). Thereafter, the cells were incubated with serum free RPMI-medium (RPMI-SF) comprising the experimental supplements in five flasks each (i.e., five replicates each). The culture was continued at 37°C, 5% CO2 and 95% humidity for 24 hours. Thereafter the respective supernatants were removed, and the adherent cells were detached from the bottom of the plate by trypsin/EDTA (Gibco; No. 25300-054) and were pelleted. After resuspension and homogenization, the cells were counted using a hemocytometer. Cultured LNCaP cells were incubated without and with 10 nM DHT (Dihydrotestosterone, in 0.09%DMSO) and increasing concentration of ethyl pyruvate (A) or pyruvate (B). The number of vital cells were counted by trypan blue staining.

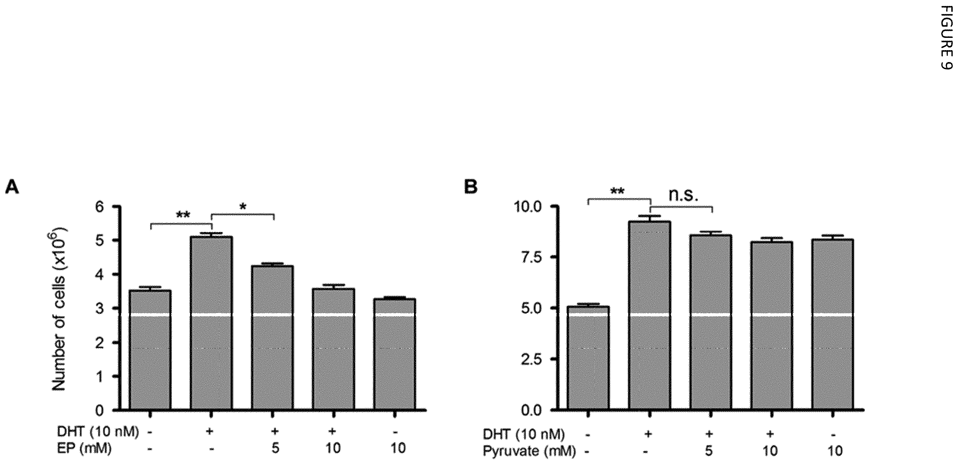

The experiment (see **Figure 9**) shows that metabolic activation of prostate cancer cells by the hormone DHT increases proliferation and are inhibited by ethyl pyruvate while non-activated cancer cells are not inhibited by ethyl pyruvate (A). Surprisingly, no inhibitory effect of pyruvate on growth-activated cells was observed (B).

## Claims

1. Ethyl pyruvate for use in the treatment or prophylaxis of influenza.

2. A pharmaceutical composition comprising ethyl pyruvate for use in the treatment or prophylaxis of influenza.

3. The pharmaceutical composition for use according to claim 2, which comprises one or more additional therapeutically active ingredients, preferably an antiviral compound, an anti-inflammatory compound, an anti-bacterial agent and/or analgesic.

4. The pharmaceutical composition for use according to claim 2 or 3, which is for topical, systemic, percutaneous, oral, intravenous, intranasal, or pulmonary administration, or for administration as an aerosol or as a spray.

5. The pharmaceutical composition for use according to claim 4, wherein said pharmaceutical composition is to be administered via the respiratory tract and/or mouth, preferably via an inhaler device or a spraying device.

6. The pharmaceutical composition for use according to any one of claims 2 to 5, wherein said pharmaceutical composition is to be administered within 48 h of the onset of at least one sign or symptom of said disease, preferably within 40 h, 36 h, 30 h, 24 h, more preferably less than 24 h of the onset of at least one sign or symptom of said disease.

7. The pharmaceutical composition for use according to any one of claims 2 to 6, wherein said pharmaceutical composition is to be administered for 2 days or more, preferably for 2 to 14 days.

8. The compound for use according to claim 1, or the pharmaceutical composition for use according to any one of claims 2 to 7, wherein at least one sign or symptom of influenza is one or more of fever or feeling feverish, chill, cough, sore throat, runny or stuffy nose, sneezing, muscle or body ache, headache, fatigue, vomiting, diarrhea, respiratory tract infection, chest discomfort, shortness of breath, bronchitis, and pneumonia.

9. The compound for use according to claim 1 or 8, or the pharmaceutical composition for use according to any one of claims 2 to 8, wherein said influenza is caused by an influenza A, B, C or D virus, or by an influenza virus which is a type H1N1, H1N2, H1N3, H1N8, H2N1, H2N2, H2N3, H3N1, H3N2, H3N8, H4N1, H5N1-H5N9, H9N2, H6N1, H7N1-H7N9, H10N8, H9N2, H10N7, H10N8, H11N2, H11N9, H17N10, H12N1, H13N2, H13N9, H14Nx, H15Nx, H16N3, H17N10, or H18N11 influenza virus.

## Patentansprüche

1. Ethylpyruvat zur Verwendung bei der Behandlung oder Prophylaxe von Influenza.

2. Pharmazeutische Zusammensetzung, umfassend Ethylpyruvat, zur Verwendung bei der Behandlung oder Prophylaxe von Influenza.

3. Pharmazeutische Zusammensetzung zur Verwendung gemäß Anspruch 2, umfassend einen oder mehrere zusätzliche therapeutisch wirksame Inhaltsstoffe, vorzugsweise eine antivirale Verbindung, eine entzündungshemmende Verbindung, ein antibakterielles Mittel und/oder ein Analgetikum.

4. Pharmazeutische Zusammensetzung zur Verwendung gemäß Anspruch 2 oder 3, die zur topischen, systemischen, perkutanen, oralen, intravenösen, intranasalen oder pulmonalen Verabreichung oder zur Verabreichung als Aerosol oder Spray bestimmt ist.

5. Pharmazeutische Zusammensetzung zur Verwendung gemäß Anspruch 4, wobei die pharmazeutische Zusammensetzung über die Atemwege und/oder den Mund, vorzugsweise über eine Inhalationsvorrichtung oder eine Sprühvorrichtung, zu verabreichen ist.

6. Pharmazeutische Zusammensetzung zur Verwendung gemäß einem der Ansprüche 2 bis 5, wobei die pharmazeutische Zusammensetzung innerhalb von 48 Stunden nach Auftreten mindestens eines Zeichens oder Symptoms der Krankheit, vorzugsweise innerhalb von 40 Stunden, 36 Stunden, 30 Stunden, 24 Stunden, noch bevorzugter innerhalb von weniger als 24 Stunden nach Auftreten mindestens eines Zeichens oder Symptoms der Krankheit zu verabreichen ist.

7. Pharmazeutische Zusammensetzung zur Verwendung gemäß einem der Ansprüche 2 bis 6, wobei die pharmazeutische Zusammensetzung 2 Tage oder länger, vorzugsweise 2 bis 14 Tage, zu verabreichen ist.

8. Verbindung zur Verwendung gemäß Anspruch 1 oder pharmazeutische Zusammensetzung zur Verwendung gemäß einem der Ansprüche 2 bis 7, wobei mindestens ein Anzeichen oder Symptom der Influenza eines oder mehrere der folgenden ist/sind: Fieber oder Fiebergefühl, Schüttelfrost, Husten, Halsschmerzen, laufende oder verstopfte Nase, Niesen, Muskel- oder Körperschmerzen, Kopfschmerzen, Müdigkeit, Erbrechen, Durchfall, Infektion der Atemwege, Brustbeschwerden, Kurzatmigkeit, Bronchitis und Lungenentzündung.

9. Verbindung zur Verwendung gemäß Anspruch 1 oder 8 oder pharmazeutische Zusammensetzung zur Verwendung gemäß einem der Ansprüche 2 bis 8, wobei Influenza durch ein Influenza-A-, -B-, -C- oder -D-Virus oder durch ein Influenzavirus vom Typ H1N1, H1N2, H1N3, H1N8, H2N1, H2N2, H2N3, H3N1, H3N2, H3N8, H4N1, H5N1-H5N9, H9N2, H6N1, H7N1-H7N9, H10N8, H9N2, H10N7, H10N8, H11N2, H11N9, H17N10, H12N1, H13N2, H13N9, H14Nx, H15Nx, H16N3, H17N10 oder H18N11 verursacht wird.

## Revendications

1. Pyruvate d'éthyle destiné à être utilisé dans le traitement ou la prophylaxie de l'influenza.

2. Composition pharmaceutique comprenant du pyruvate d'éthyle destiné à être utilisé dans le traitement ou la prophylaxie de l'influenza.

3. Composition pharmaceutique destinée à être utilisée selon la revendication 2, qui comprend un ou plusieurs ingrédients thérapeutiques actifs supplémentaires, de préférence un composé antiviral, un composé anti-inflammatoire, un agent antibactérien et/ou un analgésique.

4. Composition pharmaceutique destinée à être utilisée selon la revendication 2 ou 3, qui est destinée à une administration topique, systémique, percutanée, orale, intraveineuse, intranasale ou pulmonaire, ou à une administration sous forme d'aérosol ou de spray.

5. Composition pharmaceutique destinée à être utilisée selon la revendication 4, dans laquelle ladite composition pharmaceutique doit être administrée par les voies respiratoires et/ou la bouche, de préférence à l'aide d'un inhalateur ou d'un pulvérisateur.

6. Composition pharmaceutique destinée à être utilisée selon l'une quelconque des revendications 2 à 5, dans laquelle ladite composition pharmaceutique doit être administrée dans les 48 heures suivant l'apparition d'au moins un signe ou symptôme de ladite maladie, de préférence dans les 40 heures, 36 heures, 30 heures, 24 heures, de préférence moins de 24 heures après l'apparition d'au moins un signe ou symptôme de ladite maladie.

7. Composition pharmaceutique destinée à être utilisée selon l'une quelconque des revendications 2 à 6, dans laquelle ladite composition pharmaceutique doit être administrée pendant 2 jours ou plus, de préférence pendant 2 à 14 jours.

8. Composé destiné à être utilisé selon la revendication 1, ou la composition pharmaceutique destinée à être utilisée selon l'une quelconque des revendications 2 à 7, dans laquelle au moins un signe ou symptôme de l'influenza est un ou plusieurs des symptômes suivants : fièvre ou sensation de fièvre, frissons, toux, mal de gorge, écoulement nasal ou congestion nasale, éternuements, douleurs musculaires ou corporelles, maux de tête, fatigue, vomissements, diarrhée, infection des voies respiratoires, gêne thoracique, essoufflement, bronchite et pneumonie.

9. Composé destiné à être utilisé selon la revendication 1 ou 8, ou la composition pharmaceutique destinée à être utilisée selon l'une quelconque des revendications 2 à 8, dans lequel ladite influenza est causée par un virus de l'influenza A, B, C ou D, ou par un virus de l'influenza de type H1N1, H1N2, H1N3, H1N8, H2N1, H2N2, H2N3, H3N1, H3N2, H3N8, H4N1, H5N1-H5N9, H9N2, H6N1, H7N1-H7N9, H10N8, H9N2, H10N7, H10N8, H11N2, H11N9, H17N10, H12N1, H13N2, H13N9, H14Nx, H15Nx, H16N3, H17N10 ou H18N11.
